# EUROPEAN PATENT APPLICATION

(11) **EP 4 663 181 A1**
(43) Date of publication of application: **17.12.2025**
(21) Application number: 22899060.2
(22) Date of filing: 24.11.2022
(51) Int. Cl.: A61K 9/00, A61K 47/44, A61K 47/26, A61K 47/36, A61K 47/20, A61K 47/22, A61K 38/29, A61P 19/10, A61M 37/00

(54) **MICRONEEDLE COMPRISING PEPTIDE-BASED ACTIVE INGREDIENT AND FORMULATION COMPOSITION FOR MANUFACTURING SAME**

(30) Priority: 29.11.2021 KR 20210167529
(71) Applicant: QuadMedicine, Inc., Seongnam-si, Gyeonggi-do 13209 (KR)
(72) Inventor: BAEK, Seung Ki, Seoul 05266 (KR); CHOI, Seong O, Namyangju-si Gyeonggi-do 12276 (KR); JUN, Hye Sun, Seongnam-si Gyeonggi-do 13428 (KR); JO, Won Jun, Seongnam-si Gyeonggi-do 13175 (KR)
(74) Representative: Prüfer & Partner mbB Patentanwälte · Rechtsanwälte
(86) International application number: PCT/KR2022/018733
(87) International publication number: WO 2023/096378

(57) **Abstract**

The present invention relates to a microneedle containing a peptide-based drug and a microneedle formulation composition for manufacturing the microneedle. In particular, the microneedle formulation composition includes a biodegradable polymer; a peptide-based drug; an oil; a stabilizer; an antioxidant; and a surfactant. According to the present invention, the microneedle containing a peptide-based drug can be manufactured in a sophisticated shape such that it can permeate into a subject. Accordingly, the peptide-based drug can be rapidly delivered, and problems such as skin irritation that are problematic in the transdermal delivery of active ingredients can be solved.

## Description

### [Technical Field]

The present invention relates to a microneedle containing a peptide-based active ingredient and a formulation composition for manufacturing the same. In particular, the present invention includes a microneedle capable of rapidly delivering a peptide-based active ingredient through the skin, and a formulation composition containing a peptide-based active ingredient and being capable of manufacturing a microneedle having a precise shape.

### [Background Art]

Osteoporosis refers to a condition in which the bone content in the human body decreases and the bone strength becomes weak, which increases the possibility of fractures. In particular, women account for 94% of all osteoporosis patients, and among them, women in their 50s to 70s who have reached menopause account for the majority. The reason for the high number of female patients in their 50s or more is the lack of estrogen after menopause. Estrogen plays a role in reducing bone resorption and promoting bone formation, but estrogen levels drop sharply, causing many cases in female patients in their 50s or more.

These osteoporosis treatments are largely divided into bone resorption inhibitors and bone formation promoters. Among these treatments, bone resorption inhibitors include bisphosphonate, estrogen/antagonists, calcitonin, RANKL inhibitors, calcium preparations, vitamin preparations, and the like, and bone formation promoters include a parathyroid hormone. In particular, parathyroid hormones are currently the only osteoporosis treatment drugs that can suppress osteoblast apoptosis and increase osteoblast differentiation and reactivation, thereby resolving the fundamental cause of osteoporosis, and, accordingly, are being used as a main treatment.

As examples of these parathyroid hormones, Teriferatide, which is an injectable product manufactured by Forteo, can be administered subcutaneously once a day at 20 µg, and in Korea, Teribon can be administered subcutaneously once a week at 60 µg at a hospital. Teriferatide-based products on the market have problems such as the possibility of infection by syringe needles due to syringe use, continuous administration for 18 to 24 months, and decreased compliance with medication due to patient pain and fear of needles.

A transdermal drug delivery method is one of methods that can improve patient compliance and continuation of drug administration. A transdermal drug delivery method is largely divided into a passive transdermal drug delivery system and an active transdermal drug delivery system. The passive transdermal drug delivery system is a passive method that depends on the physicochemical properties of a drug, and is applied in the form of a cream, a patch, an ointment, or the like to the skin. Meanwhile, parathyroid hormones and synthetic preparations with the same pharmaceutical functions as in the preparations have a molecular weight of 4118 Da, which is much larger than 500 Da as a molecular weight of a drug that can be passively delivered through the skin, making it almost difficult to penetrate the skin. A microneedle, one of active transdermal drug delivery methods capable of solving the skin penetration limit, physically penetrates the stratum corneum with a thickness of 10 to 30 µm to deliver active ingredients. Examples of this active transdermal drug delivery method include a solid microneedle with a cream formulation, a microneedle coated with active ingredients, a meltable microneedle that can be dissolved in water, a hollow microneedle with a reduced size compared to existing syringes, etc.

Meanwhile, a coated microneedle can deliver active ingredients into the skin in a short time, but has a limitation in the amount of delivery. A meltable microneedle has problems such as uncertain quantitative delivery and delayed penetration time in delivering active ingredients into the skin. Therefore, there is a need for a technology that can effectively and accurately deliver active ingredients of a microneedle into the skin in a rapid manner.

### [Disclosure]

### [Technical Problem]

Therefore, the present invention has been made in view of the above problems, and it is one object of the present invention to provide a microneedle that can easily and efficiently deliver a peptide-based active ingredient to a subject and has separability and excellent solubility.

It is another object of the present invention to provide a microneedle formulation composition containing a peptide-based active ingredient and being capable of implementing a sophisticated microneedle shape that can permeate into a subject.

### [Technical Solution]

In accordance with an aspect of the present invention, the above and other objects can be accomplished by the provision of a microneedle formulation composition, including: a biodegradable molecule; a peptide-based drug; an oil; a stabilizer; an antioxidant; and a surfactant, wherein the microneedle formulation composition is provided in a gel form.

In addition, in the microneedle formulation composition of the present invention, the biodegradable polymer may be included in a ratio of 5 wt.% to 20 wt.% based on the total weight of the composition.

In addition, in the microneedle formulation composition of the present invention, the oil may include at least one selected from the group consisting of castor oil, corn oil, olive oil, peanut oil, peppermint oil and soybean oil.

In addition, the microneedle formulation composition of the present invention may further include at least one substance selected from disaccharides such as sucrose, lactose, maltose and trehalose, polysaccharides such as dextran and cellulose, and sugar alcohols such as mannitol and sorbitol as a stabilizer.

In addition, in the microneedle formulation composition of the present invention, the antioxidant may include at least one selected from the group consisting of ethylene-diaminetetraacetic acid (EDTA), L-methionine, L-Glutamine and ascorbic acid.

In addition, in the microneedle formulation composition of the present invention, the surfactant may include at least one selected from the group consisting of polysorbate 20, polysorbate 40, polysorbate 80, sorbitan monolaurate 20 (Span 20), sorbitan monolaurate (Span 80), polyethylene glycol 300 (PEG 300) and polyethylene glycol 400 (PEG 400).

In addition, in the microneedle formulation composition of the present invention, the peptide-based drug may include a parathyroid hormone (PTH (1-34)).

In addition, in the microneedle formulation composition of the present invention, the oil may be included in a ratio of 0.1 wt.% to 5.0 wt.% based on a total weight of the composition.

In addition, in the microneedle formulation composition of the present invention, the antioxidant may be included in a ratio of 0.01 wt.% to 3.0 wt.% based on the total weight of the composition.

In addition, in the microneedle formulation composition of the present invention, the surfactant may be included in a ratio of 0.01 wt.% to 5.0 wt.% based on the total weight of the composition.

In addition, in the microneedle formulation composition of the present invention, the surfactant may include at least one selected from the group consisting of polysorbate 20, polysorbate 40, polysorbate 80, sorbitan monolaurate 20 (Span 20), sorbitan monolaurate 80 (Span 80) and polyethylene glycol 400 (PEG 400).

In addition, in the microneedle formulation composition of the present invention, the surfactant may be included in a ratio of 0.01 wt.% to 5.0 wt.% based on the total weight of the composition.

In addition, in the microneedle formulation composition of the present invention, the antioxidant may include at least one selected from the group consisting of EDTA, L-methionine, ascorbic acid and L-glutamine.

In addition, in the microneedle formulation composition of the present invention, the antioxidant may be included in a ratio of 0.01 wt.% to 3.0 wt.% based on the total weight of the composition.

In accordance with another aspect of the present invention, provided is a microneedle, including: a tip configured to supply an active ingredient into a subject and to have a shape extending in at least one direction; and a base for supporting the tip, wherein the tip includes a biodegradable polymer; a peptide-based drug as an active ingredient; an oil; a stabilizer; an antioxidant; and a surfactant.

In addition, the microneedle of the present invention may further include a guider provided inside the tip and configured to extend from the base toward the tip, wherein, when the tip is inserted into a subject, the tip is separated from the guider.

In addition, in the microneedle of the present invention, the peptide-based drug may include a parathyroid hormone (PTH (1-34)), wherein a content of the parathyroid hormone is 5 to 40 wt% based on a total weight of the tip.

In addition, the microneedle of the present invention may further include a base holder provided on an opposite surface to a surface of the base, to which the base and the tip are bonded, to provide a bonding site to which the base and an outer member can be bonded.

Further, the biodegradable polymer may be included in a ratio of 25 wt.% to 65 wt.% based on the total weight of the tip.

### [Advantageous effects]

According to an aspect of the present invention, a predetermined amount of peptide-based active ingredient can be rapidly provided to a subject by using a microneedle containing the ingredient.

In addition, by using a microneedle formulation composition according to an aspect of the present invention, a sophisticated microneedle shape containing a peptide-based active ingredient and being capable of permeating into a subject can be implemented.

### [Description of Drawings]

FIG. 1 illustrates a perspective view of a microneedle according to an embodiment of the present invention.
FIG. 2 illustrates a perspective view of a microneedle according to another embodiment of the present invention.
FIG. 3 illustrates a sectional view of a microneedle according to an embodiment of the present invention.
FIG. 4 illustrates a perspective view of a microneedle further including a base holder according to another embodiment of the present invention.
FIG. 5 illustrates images of microneedles manufactured according to examples of the present invention and comparative examples.
FIG. 6 illustrates images of microneedles having different shapes and manufactured according to an example of the present invention.
FIG. 7 illustrates in-vitro skin penetration ability and separation ability evaluation results of microneedles.
FIG. 8 illustrates a photograph taken immediately after administering the microneedle patch to a Sprague-Dawley rat, but before attaching a hydrogel patch.
FIG. 9 illustrates analysis graphs about PTH contents over time in the case of being inserted with the microneedle (MAP) and a control group (Teribone).

### [Best Mode]

The present invention can be modified in various ways and can take various forms, and specific examples are illustrated in the accompanying drawings and described in detail in the text. However, this is not intended to limit the present invention to a specific disclosure form, but should be understood to include all modifications, equivalents or substitutes included in the idea and technical scope of the present invention.

According to an aspect of the present invention, a microneedle formulation composition including a biodegradable polymer; a peptide-based drug; an oil; a stabilizer; an antioxidant; and a surfactant and provided in a gel form is provided. The microneedle formulation composition may be rapidly decomposed when it penetrates into a subject, and thus, may rapidly provide a peptide-based drug transdermally.

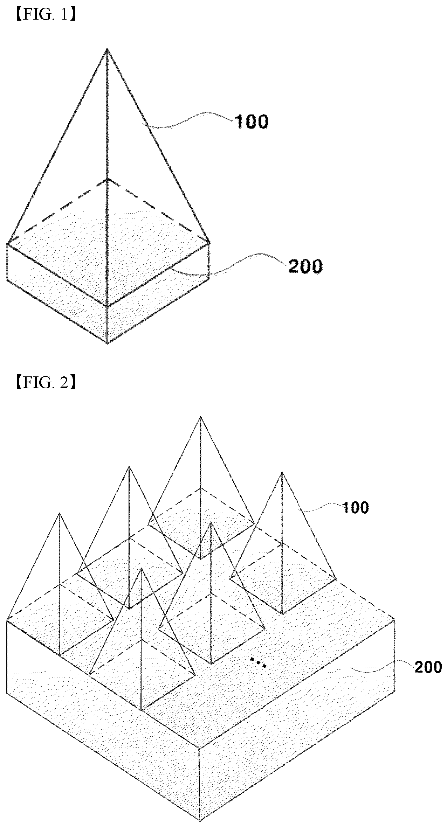

FIG. 1 illustrates a perspective view of a microneedle according to an embodiment of the present invention.

Referring to FIG. 1, the microneedle according to an embodiment of the present invention provides an active ingredient into a subject, and includes a tip 100 having a shape extending in at least one direction; and a base 200 for supporting the tip 100, wherein the tip 100 includes a biodegradable polymer; a peptide-based drug as an active ingredient; an oil; a stabilizer; an antioxidant; and a surfactant.

Hereinafter, the components are respectively described in detail.

First, the tip 100 is a member for delivering an active ingredient to a subject, and includes an active ingredient and a biodegradable polymer.

The tip 100 may have a shape extending in at least one direction. Specifically, the tip 100 may have a structure in which a length in a height direction perpendicular to the base 200 is longer than a length in a width direction parallel to the base 200. Accordingly, a relatively large pressure is applied to an end of the tip 100, and the tip 100 may easily permeate into a subject.

In some cases, the tip 100 may have a tapered shape. Accordingly, the end of the tip 100 has a narrow cross-sectional area, thereby being easy to permeate into a subject. For example, as the tip 100 has the tapered shape, it may easily permeate into the skin of a subject. The shape of the tip 100 may be, for example, a cone, a square pyramid, a triangular pyramid, etc., and various other shapes may be used in addition to these listed examples. In addition, when a plurality of tips 100 are provided, the tips 100 may be the same or different shapes.

The tip 100 includes an active ingredient and a biodegradable polymer, and, after permeating into a subject, is decomposed to release an active ingredient. The active ingredient may include a drug suitable for transdermal administration rather than oral administration. When an active ingredient (e.g., drug) is provided through transdermal administration, a constant serum drug concentration or a therapeutic target concentration may be obtained due to constant penetration of the drug. Accordingly, in the case of an active ingredient that requires continuous and constant concentration maintenance, it is advantageous to provide it through transdermal administration.

The active ingredient contained in the tip 100 may be a peptide-based drug. In general, peptide-based drugs have high molecular weights, which makes drug delivery difficult. For example, there is a problem that the molecular weight of a drug capable of passive transdermal drug delivery is 500 Da, so peptide-based drugs are almost difficult to penetrate the skin. The stratum corneum with a thickness of 10-30 µm may be physically penetrated by using a microneedle which is one of active transdermal drug delivery methods that can solve the skin penetration limit, thereby delivering an active ingredient. The tip 100 according to the present invention has the advantage of being able to deliver even a peptide-based drug with a high molecular weight, which is difficult to deliver transdermally by an existing passive transdermal drug delivery method, into the body through active transdermal drug delivery.

The peptide-based drug included in the tip 100 may include at least one of alpha-interferon, beta-interferon for multiple sclerosis, erythropoietin, follitropin beta, follitropin alpha, G-CSF, GM-CSF, human chorionic gonadotropin, luteinizing hormone, salmon calcitonin, glucagon, GNRH antagonist, insulin, human growth hormone, filgrastin, heparin, low-molecular-weight heparin and somatropin. Alternatively, the active ingredient may include a vaccine including at least one of Japanese encephalitis vaccine, rotavirus vaccine, influenza vaccine, polio vaccine, chickenpox vaccine, Alzheimer's disease vaccine, arteriosclerosis vaccine, cancer vaccine, nicotine vaccine, diphtheria vaccine, cervical cancer vaccine, meningococcal vaccine, tetanus vaccine, whooping cough vaccine, Lyme disease vaccine, rabies vaccine, pneumococcal vaccine, yellow fever vaccine, cholera vaccine, smallpox vaccine, tuberculosis vaccine, rubella vaccine, measles vaccine, mumps vaccine, botulism vaccine, herpes virus vaccine, other DNA vaccines, hepatitis B vaccine, hyaluronic acid, coenzyme Q10, chitosan, botox, vitamins and vitamin derivatives, hydroxy acid, tetracycline, oxytetracycline, doxycycline, minocycline, benzocaine, mepivacaine, lidocaine, prilocaine, bupivacaine, etidocaine, articaine, procaine, propoxycaine, tetracaine, ropivacaine, butacaine, piperocaine, cocaine, chloroprocaine, proparacaine and dyclonine.

In some cases, the tip 100 may be provided with a parathyroid hormone (PTH (1-34). Here, the parathyroid hormone may be included in a ratio of 5 wt.% to 40 wt.% in the tip 100. When the parathyroid hormone is administered in a ratio of 1 wt.% or less into a subject, the amount of the parathyroid hormone may be small, resulting in a minimal therapeutic effect. On the other hand, when the content of the parathyroid hormone is greater than 40 wt.%, it may be difficult to provide a tip 100 with sufficient strength, and the molding processability of the tip 100 may deteriorate.

The biodegradable polymer contained in the tip 100 is a material capable of maintaining the shape of the tip 100 outside a subject such that the active ingredient can be delivered inside the subject and capable of being decomposed without affecting the subject when permeated into the subject. Here, the biodegradable polymer being decomposed inside a subject may mean that the tip 100 loses its original tapered shape and dissolves. A biodegradable polymer may include at least one selected from the group consisting of carboxymethyl cellulose (CMC), hyaluronic acid (HA), polyvinylpyrrolidone (PVP), hydroxypropyl methyl cellulose (HPMC), ethyl cellulose (EC) and hydroxypropyl cellulose (HPC).

In the microneedle formulation composition constituting the tip 100, a biodegradable polymer may be included in a ratio of 5 wt.% to 20 wt.% based on the total weight of the composition. When the content of the biodegradable polymer is less than 5 wt.%, the concentration of the composition is low, so that sufficient strength may not be obtained, and since the composition is provided in a liquid state rather than a gel form, molding processability may deteriorate. On the other hand, when the content of the biodegradable polymer in the composition is greater than 20 wt.%, the composition is changed into a powder form rather than a smooth gel form, making it difficult to fill in the mold, and thus making it difficult to manufacture microneedles.

An intrinsic viscosity of the biodegradable polymer material in the microneedle formulation composition constituting the tip 100 may be 0.5 m3/kg to 3.3 m3/kg. When the intrinsic viscosity is less than 0.5 m3/kg, the mechanical strength required for skin penetration may not be provided. In addition, when the intrinsic viscosity is greater than 3.3 m3/kg, the viscosity of the composition is too high, making it difficult to uniformly fill the composition in the mold.

The biodegradable polymer in the microneedle formulation composition constituting the tip 100 may have an average molecular weight of 500 kDa to 3,000 kDa. When using a biodegradable polymer with the molecular weight range, appropriate intrinsic viscosity may be secured, and biodegradability with an appropriate speed may be provided.

At least some regions of the tip 100 may be coated with a waterproofing agent. The waterproofing agent prevents the tip 100 from being damaged by moisture in the air before it permeates into a subject. The waterproofing agent may include at least one of beeswax, oleic acid, soy fatty acid, castor oil, phosphatidylcholine, vitamin E (d-α-tocopherol/vitamin E), corn oil, mono-di-tridiglycerides, cottonseed oil, olive oil, peanut oil, peppermint oil, safflower oil, sesame oil, soybean oil, hydrogenated vegetable oils, hydrogenated soybean oil, caprylic/capric triglycerides derived from coconut oil or palm seed oil and phosphatidylcholine, or may be formed of a mixture thereof.

The tip 100 includes an oil. The oil may include at least one selected from the group consisting of castor oil, corn oil, olive oil, peanut oil, peppermint oil and soybean oil.

An oil in the formulation composition constituting the tip 100 may be included in a ratio of 0.1 wt.% to 5 wt.% based on the total weight of the composition. When the oil is included in a ratio of less than 0.1 wt.%, wettability between the surface of mold and the composition filled in the mold is reduced, so that, when the formulation composition is dried, the shape of a needle may not be properly formed. On the other hand, when the oil is included in a ratio of greater than 5 wt.%, it may be difficult to secure sufficient viscosity of the composition, and an oil portion and a water-soluble portion may be separated from each other during the manufacturing process of the tip 100.

In addition, the tip includes a surfactant. The surfactant may include at least one selected from the group consisting of polysorbate 20, polysorbate 40, polysorbate 80, sorbitan monolaurate 20 (Span 20), sorbitan monolaurate 80 (Span 80) and polyethylene glycol 400 (PEG 400).

The surfactant may be included in a ratio of 0.01 wt.% to 5.0 wt.% based on the total weight of the composition. When the surfactant is included in a ratio of less than 0.01 wt.%, phase separation may occur between the oil portion and the water-soluble portion. On the other hand, when the surfactant is included in a ratio of greater than 5 wt.%, a lot of bubbles may occur during the mixing of the formulation composition, which may cause uneven mold filling during subsequent microneedle manufacturing.

In addition, the tip includes an antioxidant. The antioxidant may include at least one selected from the group consisting of EDTA, L-methionine, ascorbic acid and L-glutamine.

Depending upon the types of the antioxidant, the antioxidant may be included in a ratio of 0.01 wt.% to 3.0 wt.% based on the total weight of the composition. In particular, in the case of PTH (1-34), oxidation of methionine located at amino acid sequence numbers 8 and 18 occurs very easily. EDTA, one of antioxidants for suppressing this, is a chelating agent that neutralizes metals capable of promoting autooxidation reactions and thus inhibits oxidation reactions. Another type is L-methionine, a free radical scavenger, which neutralizes reactive oxygen species and inhibits oxidation reactions. As other antioxidants, there are L-glutamine, ascorbic acid, and the like.

The tip 100 is supported by the base 200.

The base 200 is a body that supports the tip 100, and may be attached to the subject while supporting the tip 100. The base part 200 is preferably low reactivity because it adheres to the subject without permeating into the subject. For example, the base 200 may be manufactured from a material that does not dissolve in water and does not cause an inflammatory reaction, etc.

The base 200 may be manufactured from at least one of, for example, polyethylene (PE), polypropylene (PP), polytetrafluoroethylene (PTFE), polymethyl methacrylate (PMMA), ethylene vinyl acetate (EVA), polycaprolactone (PCL), polyurethane (PU), polyethylene terephthalate (PET), polyethylene glycol (PEG), polyvinyl alcohol (PVA), polylactide (PLA), polylactide-glycolide copolymer (PLGA), and polyglycolide (PGA).

The base 200 may have an area larger than the bottom area of the tip 100 because it supports the tip 100. There is no limitation on the shape of the base 200, and it may have various shapes such as a square, trapezoid, circle, ellipse, triangle, etc. on a plane.

In some cases, the base 200 may have flexibility. Accordingly, when the base part 200 is in close contact with the surface of a subject, the base part 200 may be bent or folded along the curvature of the subject surface. Accordingly, even if the subject surface is curved, the base 200 may be provided in close contact with the subject surface, and the tip 100 may be permeated into the subject at a uniform depth.

FIG. 2 illustrates a perspective view of a microneedle according to another embodiment of the present invention.

Referring to FIG. 2, the microneedle includes a plurality of tips 100. The plural tips 100 may be arranged on a base 200 in a plurality of columns and/or a plurality of rows.

The plural tips 100 may be separated from each other. A distance between the tip 100 may be determined in consideration of the height and cross-sectional area of the tip 100. For example, the tip 100 may have a height of about 50 µm to about 2000 µm from the base 200. Here, a distance between the tips 100 may be in a range of about 30% to about 200% of the height of the tips 100.

Here, the base 200 may have a large area to support the plural tips 100. The shape of the base 200 is not limited so long as it can support the plural tips 100.

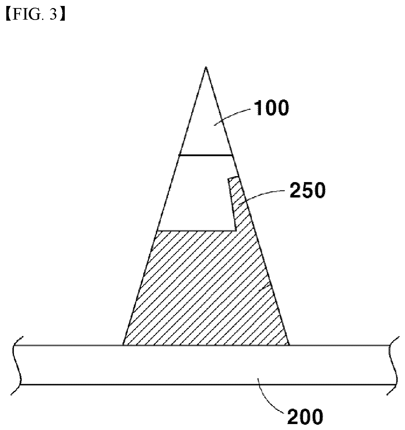

FIG. 3 illustrates a sectional view of a microneedle according to an embodiment of the present invention.

The microneedle of FIG. 3 further includes a guider 250 that is provided inside the tip 100 and extends from the base 200 toward the tip 100. When the tip 100 is inserted into a subject, the tip 100 is separated from the guider 250.

The guider 250 may have a shape that protrudes in a penetration direction in which the tip 100 permeates a subject. When a plurality of tips 100 are provided, a plurality of guiders 250 may also be provided such that the guiders 250 are respectively coupled to the plural tips 100. The guider 250 is provided between the base 200 and the tip 100 to be coupled to a rear end of the tip 100 rather than a pointed tip end thereof. That is, the guider 250 may have a shape that extends from the base 200 toward the tip 100.

In some cases, the guider 250 may further include a plurality of guider protrusions provided on the outer peripheral surface thereof. As the guider protrusions are provided, the bonding force between the tip 100 and the guider 250 may be further reduced. The guider protrusion supports the tip 100 extending in the penetration direction and guides the permeation of the tip 100. Here, the guider protrusions may be provided integrally with the guider 250 extending from the base 200.

The geometric structure of the guider 250 is characterized by protruding into the tip 100 to protect the tip 100 from damage due to a force applied in a horizontal direction, thereby ensuring stability in the vertical direction. This guider 250 may have a shape protruding into the tip 100 to correspond to the tip 100 having a square pyramid shape as shown in the drawing, but is not limited thereto and various embodiments are possible. The guider 250 may have a shape wherein the guider protrusions protrude from one of the sides of the guider 250 or from two to four side surfaces thereof, a shape wherein the guider 250 is formed as a flat surface and the guider protrusions protrude from the upper surface of the guider 250, etc.

The guider 250 may lower the bonding force between the tip 100 and the base 200. Accordingly, the bonding force between the tip 100 and a subject may be greater than the bonding force between the guider 250 and the tip 100. Specifically, the guider 250 may guide separation by controlling the bonding force between the guider 250 and the tip 100 based on the geometric structure protruding from the base 200. A bonding force range between the tip 100 and the guider 250 may be approximately 0.0001N to 1N or less, and the sum of the elasticity, friction, and adhesive force of the skin S, which is the force that a subject (skin, etc.) uses to hold the tip 100, may be 1N to 5N. Accordingly, since the force of the skin S holding the tip 100 is relatively greater than the bonding force between the tip 100 and the guider 250, the tip 100 that has permeated the skin S may be immediately separated from the guider 250.

One of the biggest drawbacks of transdermal drug delivery is the potential for local irritation at the site of application. Skin flushing, itching, localized swelling, etc. may be caused by other factors such as patch-type drug formulations and adhesives. For most patients, irritation can be reduced by position rotation. However, some patients may develop severe allergic reactions to transdermal patches. In these cases, treatment should be discontinued. Accordingly, in the case of a microneedle that delivers an active material transdermally, it is desirable to rapidly remove the active material from the subject after rapidly delivering the active material. In the present invention, as the guider 250 is provided as described above, the tip 100 may be immediately separated and provided into the subject, and then the microneedle may be removed from the subject. Accordingly, the stimulation due to the microneedle is reduced.

The guider 250 may be made of an insoluble material, unlike the tip 100 permeating into a subject. Accordingly, it does not interfere with the penetration power of the tip 100 to be melted, so it is possible to guide the supply of a predetermined amount of medicine into the skin S.

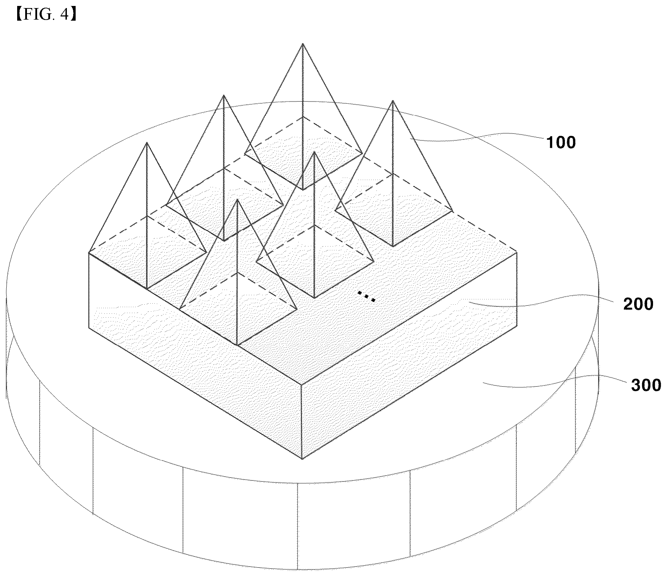

FIG. 4 illustrates a perspective view of a microneedle further including a base holder according to another embodiment of the present invention.

A base holder 300 is provided on an opposite surface to a surface of the base 200 where the base 200 and the tips 100 are bonded, thereby providing a bonding site where the base 200 and an outer member can be bonded. The base holder 300 may be detachably connected to the outer member. Accordingly, usability is increased because a plurality of microneedles loaded with different active materials can be provided simultaneously and the type of microneedle can be changed as needed.

The microneedle according to an embodiment of the present invention has been examined above. Hereinafter, the advantageous effects of the microneedle of the present invention will be examined by comparing examples and comparative examples.

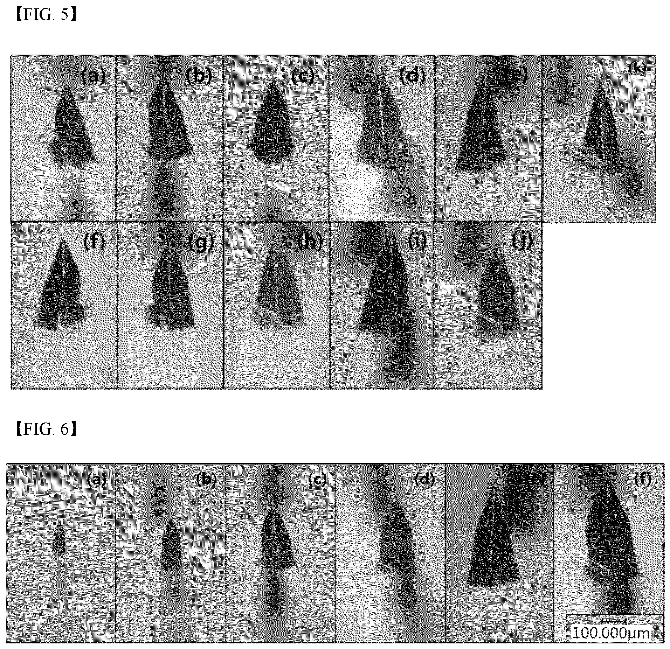

FIG. 5 illustrates images of microneedles manufactured according to examples of the present invention and comparative examples.

The microneedles of FIG. 5 were manufactured using formulation compositions of Preparation Examples 1 to 5 below.

### Preparation Example (1) - Preparation of gel-type microneedle formulation compositions dependent upon concentrations and extreme viscosities of biodegradable polymers

In Preparation Example (1), formulation compositions of examples and comparative examples in which the concentrations and extreme viscosities of biodegradable polymers were different were prepared. In addition, a small amount of gentian violet was added to make the tip appear purple, making it easier to observe the needle properties.

**[Table 1]**

| | Biodegradable polymer (w/w) | PTH (w/w) | Stabilizer (w/w) | Oil (w/w) | Antioxidant (w/w) | Surfactant (w/w) |
|---|---|---|---|---|---|---|
| Comparative Example 1-1 | Extreme viscosity 1.6 m3/kg used, 1% | 4% | Sucrose-10% | Castor oil, 1% | EDTA, 0.15% | Tween 20, 0.4% |
| Example 1-1 | Extreme viscosity 1.6 m3/kg used, 5% | 4% | Sucrose-10% | Castor oil, 1% | EDTA, 0.15% | Tween 20, 0.4% |
| Example 1-2 | Extreme viscosity 1.6 m3/kg used, 10% | 4% | Sucrose-10% | Castor oil, 1% | EDTA, 0.15% | Tween 20, 0.4% |
| Example 1-3 | Extreme viscosity 1.6 m3/kg used, 15% | 4% | Sucrose-10% | Castor oil, 1% | EDTA, 0.15% | Tween 20, 0.4% |
| Example 1-4 | Extreme viscosity 1.6 m3/kg used, 20% | 4% | Sucrose-10% | Castor oil, 1% | EDTA, 0.15% | Tween 20, 0.4% |
| Comparative Example 1-2 | Extreme viscosity 1.6 m3/kg used, 25% | 4% | Sucrose-10% | Castor oil, 1% | EDTA, 0.15% | Tween 20, 0.4% |
| Comparative Example 1-3 | Extreme viscosity 0.5 m3/kg used, 20% | 4% | Sucrose-10% | Castor oil, 1% | EDTA, 0.15% | Tween 20, 0.4% |
| Comparative Example 1-4 | Extreme viscosity 3.0 m3/kg used, 10% | 4% | Sucrose-10% | Castor oil, 1% | EDTA, 0.15% | Tween 20, 0.4% |

Microneedles were manufactured using the formulation compositions of the examples and the comparative examples according to Preparation Example 1. As a result, even when the extreme viscosity and content of hyaluronic acid were changed within the ranges of the examples, this did not significantly affect the manufacture of microneedles (FIG. 5(a) and (b)).

However, in the case of Comparative Example 1-1, the content of hyaluronic acid was too low, so there was a limit in forming the shape of the needle (FIG. 5(k)). In addition, in the case of Comparative Examples 1-2 to 1-4, the content of hyaluronic acid was very high, so that a snow-white powder-like shape, not a smooth gel shape, was observed, making filling into a mold difficult and thus unsuitable for producing microneedles.

### Preparation Example (2) - Evaluation of preparation of gel-type microneedle formulation compositions dependent upon type or presence/absence of oil

Formulation compositions were prepared by adding hyaluronic acid, which is a biodegradable polymer and has an average molecular weight of 1 million Da, PTH (1-34), which is a parathyroid hormone, sucrose used as a stabilizer, EDTA, which is one of antioxidants, and Tween 20, which is one of surfactants, to purified water in the amounts shown in Table 2 below and adding different types and concentrations of oil. In addition, A small amount of gentian violet was added to make the tips purple, making it easier to observe the needle shapes.

**[Table 2]**

| | Biodegradable polymer (w/w) | PTH (w/w) | Stabilizer (w/w) | Oil (w/w) | Antioxidant (w/w) | Surfactant (w/w) |
|---|---|---|---|---|---|---|
| Example 2-1 | 10% | 4% | Sucrose-10% | Castor oil, 0.1% | EDTA, 0.15% | Tween 20, 0.4% |
| Example 2-2 | 10% | 4% | Sucrose-10% | Castor oil, 1% | EDTA, 0.15% | Tween 20, 0.4% |
| Example 2-3 | 10% | 4% | Sucrose-10% | Castor oil, 3% | EDTA, 0.15% | Tween 20, 0.4% |
| Example 2-4 | 10% | 4% | Sucrose-10% | Castor oil, 5% | EDTA, 0.15% | Tween 20, 0.4% |
| Comparative Example 2 | 10% | 4% | Sucrose-10% | N/A | EDTA, 0.15% | Tween 20, 0.4% |
| Example 2-5 | 10% | 4% | Sucrose-10% | Corn oil, 1% | EDTA, 0.15% | Tween 20, 0.4% |
| Example 2-6 | 10% | 4% | Sucrose-10% | Olive oil, 1% | EDTA, 0.15% | Tween 20, 0.4% |

When producing microneedles using the formulation compositions of Preparation Example 2, microneedle production was not significantly affected when the concentrations and types of oils were changed within the scope of the examples (FIG. 5(c) and (d)). However, in the case of the compositions of the comparative examples, an oil component was not contained, so that the wettability to the mold surface was low, whereby the shapes of the needles were not properly formed when the formulation compositions were dried.

### Preparation Example (3) - Preparation of gel-type microneedle formulation compositions dependent upon types and concentrations of saccharides

Formulation compositions were prepared by adding hyaluronic acid, which is a biodegradable polymer and has an average molecular weight of 1 million Da, PTH (1-34), which is a parathyroid hormone, a castor oil, which is one oil type, EDTA, which is one of antioxidants, and Tween 20, which is one of surfactants, to purified water in the amounts shown in Table 3 below and adding different types and concentrations of saccharides. In addition, A small amount of gentian violet was added to make the tips purple, making it easier to observe the needle shapes.

**[Table 3]**

| | Hyaluronic acid (w/w) | PTH (w/w) | Stabilizer (saccharide, w/w) | Oil (w/w) | Antioxidant (w/w) | Surfactant (w/w) |
|---|---|---|---|---|---|---|
| Comparative Example 3 | 10% | 4% | N/A | Castor oil, 1% | EDTA, 0.15% | Tween 20, 0.4% |
| Example 3-1 | 10% | 4% | Sucrose-8% | Castor oil, 1% | EDTA, 0.15% | Tween 20, 0.4% |
| Example 3-2 | 10% | 4% | Sucrose-15% | Castor oil, 1% | EDTA, 0.15% | Tween 20, 0.4% |
| Example 3-3 | 10% | 4% | Trehalose-10% | Castor oil, 1% | EDTA, 0.15% | Tween 20, 0.4% |
| Example 3-4 | 10% | 4% | Maltose-10% | Castor oil, 1% | EDTA, 0.15% | Tween 20, 0.4% |
| Example 3-5 | 10% | 4% | Maltodextrin-10% | Castor oil, 1% | EDTA, 0.15% | Tween 20, 0.4% |
| Example 3-6 | 10% | 4% | Lactose -10% | Corn oil, 1% | EDTA, 0.15% | Tween 20, 0.4% |

When manufacturing microneedles using the formulation compositions of Example 3, the types of saccharides did not significantly affect the manufacturing of microneedle patches (FIG. 5 (e) and (f)). Accordingly, it is possible to select saccharides that can improve the stability of the PTH drug. However, when saccharide was not contained, the formulation stability of the composition for manufacturing a microneedle was reduced.

### Preparation Example (4) - Preparation of gel-type microneedle formulation compositions dependent upon types and presence/absence of surfactants

Formulation compositions were prepared by adding hyaluronic acid, which is a biodegradable polymer and has an average molecular weight of 1 million Da, PTH (1-34), which is a parathyroid hormone, sucrose, which is a stabilizer, a castor oil, which is one oil type, and EDTA, which is one of antioxidants to purified water in the amounts shown in Table 4 below and adding different types and concentrations of surfactants. In addition, A small amount of gentian violet was added to make the tips purple, making it easier to observe the needle shapes.

**[Table 4]**

| | Hyaluronic acid (w/w) | PTH (w/w) | Stabilizer (w/w) | Oil (w/w) | Antioxidant (w/w) | Surfactant (w/w) |
|---|---|---|---|---|---|---|
| Example 4-1 | 10% | 4% | Sucrose 10% | Castor oil, 1% | EDTA, (0.15%) | Tween 20, 0.01% |
| Example 4-2 | 10% | 4% | Sucrose-10% | Castor oil, 1% | EDTA, (0.15%) | Tween 20, 0.1% |
| Example 4-3 | 10% | 4% | Sucrose-15% | Castor oil, 1% | EDTA, (0.15%) | Tween 20, 1% |
| Example 4-4 | 10% | 4% | Sucrose-15% | Castor oil, 1% | EDTA, (0.15%) | Tween 20, 2.5% |
| Example 4-5 | 10% | 4% | Sucrose-15% | Castor oil, 1% | EDTA, (0.15%) | Tween 20, 5% |
| Example 4-6 | 10% | 4% | Sucrose-10% | Castor oil, 1% | EDTA, (0.15%) | Tween 80, 0.05% |
| Example 4-7 | 10% | 4% | Sucrose-10% | Castor oil, 1% | EDTA, (0.15%) | Tween 80, 1% |
| Example 4-8 | 10% | 4% | Sucrose-10% | Castor oil, 1% | EDTA, (0.15%) | Span 80 1% |
| Comparative Example 4 | 10% | 4% | Sucrose-10% | Castor oil, 1% | EDTA, (0.15%) | N/A |

When manufacturing microneedles using the formulation compositions of Preparation Example 4, the types and concentrations of surfactants did not significantly affect the production of microneedle patches within the range of the example (FIG. 5(g) and (h)). However, in the case of the formulation composition of Comparative Example 4 excluding a surfactant, castor oil did not form an emulsion due to a surfactant, and thus the oil component was phase-separated over time.

### Preparation Example (5) - Preparation of gel-type microneedle formulation compositions dependent upon types and presence/absence of antioxidants

Formulation compositions were prepared by adding hyaluronic acid, which is a biodegradable polymer and has an average molecular weight of 1 million Da, PTH (1-34), which is a parathyroid hormone, sucrose, which is a stabilizer, a castor oil, which is one oil type, and Tween 20, which is one of surfactants, to purified water in the amounts shown in Table 5 below and adding different types and concentrations of antioxidants. In addition, A small amount of gentian violet was added to make the tips purple, making it easier to observe the needle shapes.

**[Table 5]**

| . | Hyaluronic acid (w/w) | PTH (w/w) | Stabilizer (w/w) | Oil (w/w) | Antioxidant (w/w) | Surfactant (w/w) |
|---|---|---|---|---|---|---|
| Example 5-1 | 10% | 4% | Sucrose 10% | Castor oil, 1% | EDTA, 0.01% | Tween 20, 0.4% |
| Example 5-2 | 10% | 4% | Sucrose-10% | Castor oil, 1% | EDTA, 0.1% | Tween 20, 0.4% |
| Example 5-3 | 10% | 4% | Sucrose-15% | Castor oil, 1% | EDTA, 0.2% | Tween 20, 0.4% |
| Example 5-4 | 10% | 4% | Sucrose-10% | Castor oil, 1% | L-methionine, 3% | Tween 20, 0.4% |
| Example 5-5 | 10% | 4% | Sucrose-10% | Castor oil, 1% | L-Glutamine, (1%) | Tween 20, 0.4% |
| Example 5-6 | 10% | 4% | Sucrose-10% | Castor oil, 1% | Ascorbic acid (5%) | Tween 20, 0.4% |
| Comparative Example 5 | 10% | 4% | Sucrose-10% | Castor oil, 1% | N/A | Tween 20, 0.4% |

When manufacturing microneedles using the formulation compositions of Preparation Example 5, the types and concentrations of antioxidants did not significantly affect the manufacturing of microneedle patches within the range of the example (FIG. 5(i) and (j)) However, in the case of the formulation composition of Comparative Example 5 excluding an antioxidant, oxidation of PTH (1-34) occurred more than in the microneedle patch manufactured with a different formulation composition, resulting in a decrease in drug stability (Experimental Example 3).

Next, the possibility of manufacturing various shapes of microneedles was confirmed using the formulation composition of Example 1-1 of Preparation Example (1).

FIG. 6 illustrates images of microneedles having different shapes and manufactured according to an example of the present invention.

The heights of the microneedles shown in FIGS. 6(a) to (f) are 300 (± 15) µm, 400 (± 20) µm, 500 (± 22) µm, 600 (± 23) µm, 700(± 23) µm and 800 (± 23) µm, respectively.

As shown in the drawing, microneedles having a height of 300 µm to 800 µm may be successfully manufactured using the formulation composition of Example 1-1. Accordingly, Accordingly, a plurality of microneedles having appropriate heights may be provided as needed.

### Experimental Example (1) - Evaluation of concentration of PTH (1-34) drug in formulation composition and PTH (1-34) content in microneedle patch dependent upon height of microneedle

To prepare formulation compositions, 10% (w/w) of hyaluronic acid, which is a biodegradable polymer with an average molecular weight of 1 million Da, 7% (w/w) of sucrose, which is a stabilizer, 1% (w/w) of castor oil, which is one oil type, 0.4% (w/w) of Tween 20, which one of surfactants, and 0.15% (w/w) of EDTA, which is one of antioxidants, were added to purified water, and the concentration of PTH (1-34), which is a parathyroid hormone, was varied. In addition, using the formulation compositions, microneedle patches were manufactured using a mold with a microneedle height of each of 300 µm, 500 µm, and 700 µm. Next, the amount of PTH (1-34) contained in each of the microneedle patches was measured using reverse-phase HPLC.

**[Table 6]**

| | PTH (1-34) in formulation composition (w/w) | Height of microneedle (µm) | PTH (1-34) content (µg) in microneedle patch | Ratio (%) of PTH (1-34) compared to total weight of tips |
|---|---|---|---|---|
| Experimental Example 1-1 | 1% | 500 µm | 14 ± 1.1 µg | 4.95% |
| Experimental Example 1-2 | 2% | 500 µm | 27 ± 3.3 µg | 9.42% |
| Experimental Example 1-3 | 4% | 500 µm | 58 ± 4.8 µg | 17.10% |
| Experimental Example 1-4 | 8% | 500 µm | 104 ± 6.9 µg | 30.15% |
| Experimental Example 1-5 | 12% | 500 µm | 150 ± 8.7 µg | 40.19% |
| Experimental Example 1-6 | 4% | 300 µm | 17 ± 1.1 µg | 17.10% |
| Experimental Example 1-7 | 4% | 700 µm | 190 ± 9.2 µg | 17.10% |

As shown in Table 6, it was found that a microneedle patch can be manufactured depending upon the concentration of PTH (1-34) in the formulation composition and the height of the microneedle. In particular, it was confirmed that, as the concentration of PTH (1-34) in the formulation composition increased, the content of PTH (1-34) in the microneedle patch also increased, and, as the height of the needle increased, the content of PTH (1-34) in the microneedle patch also increased.

### Experimental Example (2) - Evaluation of mechanical strength of microneedle patch

Each of microneedle patches with different PTH (1-34) ratios relative to the total weight of the tip, manufactured in Experimental Example (1), was placed on a prepared pressure device. The mechanical strength was measured by applying a force in the axial direction with respect to the microneedle patch. The mechanical strength measurement was terminated when the tip was separated from the base.

**[Table 7]**

| | Ratio (%) of PTH (1-34) compared to total weight of tips | Height of microneedle (µm) | Force when tip is separated from base |
|---|---|---|---|
| Experimental Example 2-1 | 4.95% | 500 µm | 34 ± 1.6 N |
| Experimental Example 2-2 | 9.42% | 500 µm | 29 ± 2.4 N |
| Experimental Example 2-3 | 17.10% | 500 µm | 27 ± 2.7 N |
| Experimental Example 2-4 | 30.15% | 500 µm | 23 ± 3.3 N |
| Experimental Example 2-5 | 40.19% | 500 µm | 19 ± 1.8N |
| Experimental Example 2-6 | 51.08% | 500 µm | 7 ± 1.6 N |

As shown in Table 7, it can be seen that the mechanical strength decreases as the content of PTH (1-34) relative to the total weight of the tip increases. However, mechanical strength sufficient to penetrate the skin was observed between 5% and 40%. In addition, it was observed that the separation force was significantly reduced when the content of PTH (1-34) exceeded 40%. Accordingly, it was confirmed that it was better to set the content ratio of PTH (1-34) relative to the total weight of the tip to 5% to 40%. In addition, when exceeded 40%, the surface of the microneedle tip was very rough.

### Experimental Example (3) - Stability evaluation of PTH (1-34) drug dependent upon types and concentrations of antioxidants

To prepare formulation compositions, 10% (w/w) of a biodegradable polymer, which is hyaluronic acid having an average molecular weight of 1 million Da, 4% (w/w) of PTH (1-34) which is a parathyroid hormone, 7% (w/w) of sucrose, which is a stabilizer, 1% (w/w) of castor oil, which is one oil type, and 0.4% (w/w) of Tween 20, which is one of surfactants were added to purified water, and the types and concentration of antioxidants were varied. Using the formulation compositions, microneedle patches were manufactured. Next, the stability of PTH (1-34) contained in each of the microneedle patches was measured using reverse-phase HPLC.

**[Table 8]**

| | Antioxida nt | Time after patch production | PTH (1-34) purit y (%) | Oxidatio n 1 (%) | Oxidatio n 2 (%) | Oxidatio n 3 (%) | Total Oxidatio n (%) |
|---|---|---|---|---|---|---|---|
| Experiment al Example 3-1 | EDTA, 0.01% | Immediatel y after production | 94.9 8 | 0.00 | 0.08 | 0.18 | 0.26 |
| | | 1 month later | 93.7 6 | 0.00 | 0.09 | 0.29 | 0.38 |
| Experiment al Example 3-2 | EDTA, 0.1% | Immediatel y after production | 95.7 6 | 0.00 | 0.08 | 0.14 | 0.22 |
| | | 1 month later | 94.9 9 | 0.00 | 0.09 | 0.20 | 0.29 |
| Experiment al Example 3-3 | EDTA, 0.2% | Immediatel y after production | 95.8 1 | 0.00 | 0.07 | 0.13 | 0.20 |
| | | 1 month later | 95.5 7 | 0.00 | 0.07 | 0.16 | 0.23 |
| Experiment al Example 3-4 | ascorbic acid, 5% | Immediatel y after production | 93.5 0 | 0.00 | 0.08 | 0.17 | 0.25 |
| | | 1 month later | 92.2 9 | 0.00 | 0.1 | 0.31 | 0.41 |
| Experiment al Example 3-5 | L-methionin e, 3% | Immediatel y after production | 95.4 1 | 0.00 | 0.07 | 0.14 | 0.21 |
| | | 1 month later | 94.6 9 | 0.00 | 0.07 | 0.15 | 0.22 |
| Experiment al Example 3-6 | L-Glutamine , 1% | Immediatel y after production | 95.1 7 | 0.00 | 0.09 | 0.23 | 0.32 |
| | | 1 month later | 93.7 7 | 0.01 | 0.16 | 0.41 | 0.58 |
| Comparativ e Example 3-1 | N/A | Immediatel y after production | 91.7 6 | 0.02 | 0.14 | 0.31 | 0.47 |
| | | 1 month later | 87.2 8 | 0.08 | 0.41 | 0.59 | 1.08 |

As shown in Table 8, it was confirmed that, when the antioxidant was added, the purity of PTH (1-34) and the stability thereof against oxidization were higher than those in Comparative Example 3-1.

### Experimental Example (4) - Evaluation of in-vitro skin penetration ability and separation ability of microneedles

Frozen porcine back skin was thawed at room temperature for more than 1 hour. After sufficiently removing moisture from the thawed skin, microneedle patches of 300 µm, 500 µm, and 700 µm in height were combined with an applicator and applied to the porcine skin. After 3 seconds of application of the applicator, the microneedle patches were removed from the skin to evaluate the skin penetration ability and separation ability.

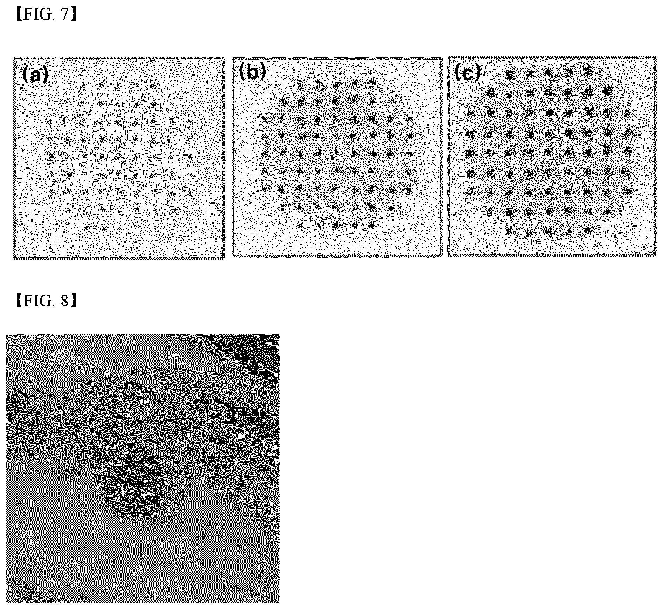

FIG. 7 illustrates in-vitro skin penetration ability and separation ability evaluation results of microneedles.

FIG. 7 shows photographs taken immediately after administering each of the microneedle patches to the porcine skin. Specifically, (a) represents the case applied with a microneedle height of 300 µm, (b) represents the case applied with a microneedle height of 500 µm, and (c) represents the case applied with a microneedle height of 700 µm, respectively.

Referring to FIG. 7(a) to (c), it can be seen from the experimental results that 100% of the microneedle tips are inserted regardless of the microneedle height.

Therefore, it was confirmed that the microneedle exhibited the skin penetration ability and permeation ability within the height range of the microneedle described above.

### Experimental Example (5) - Evaluation of in-vivo the skin penetration ability and separation ability of microneedle

The microneedle patch containing about 60 µg of PTH was inserted into the dorsal area of an 8-week-old male Sprague-Dawley rat. Immediately after insertion, the microneedle patch was removed, and then a hydrogel patch was attached. Since the hydrogel patch was transparent, the number of holes pierced due to the insertion could be counted.

FIG. 8 illustrates a photograph taken immediately after administering the microneedle patch to a Sprague-Dawley rat, but before attaching a hydrogel patch.

Referring to FIG. 8, it can be seen from the experimental result that 100% of the microneedle tip was inserted. Therefore, it was confirmed that the microneedle according to the example could sufficiently penetrate the subject's skin.

### Experimental Example (6) - PK Profile of microneedle patch containing PTH

The microneedle patch containing 60 µg of PTH (1-34) was inserted into the dorsal region of 8-week-old male Sprague-Dawley rats. In addition, Teribone injection (PTH (1-34): 56.5 µg) was injected subcutaneously for a comparative group experiment. In each experimental group, blood samples were collected at 0, 5, 10, 15, 30, 60, 120, and 240 minutes, and the blood samples obtained at each time point were centrifuged to obtain serum and subjected to content evaluation using an ELISA kit.

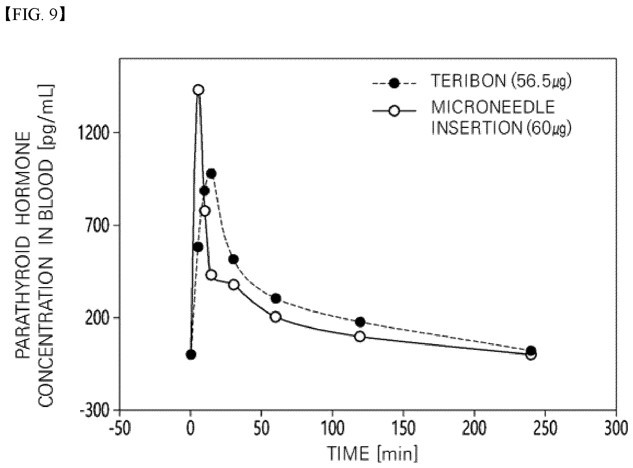

FIG. 9 illustrates analysis graphs about PTH contents over time in the case of being inserted with the microneedle (MAP) and a control group (Teribone).

Referring to FIG. 9, it can be confirmed in the case of the example that the blood PTH concentration quickly soars to 1200 pg/mL or more immediately after the microneedle patch is applied. This is a higher value than the blood concentration collected from the comparative example, Teribone, and it can be confirmed that the time required to reach the maximum concentration is also significantly shorter. Therefore, it was confirmed that delivering PTH using the microneedle can achieve a more immediate effect than conventional methods.

Although the present invention has been described above with reference to preferred embodiments thereof, it will be understood that those skilled in the art or having common knowledge of the art can modify and change the present invention in various ways without departing from the scope and technical scope of the present invention described in the accompanying claims.

Therefore, the technical scope of the present invention should not be limited to the contents described in the detailed description of this specification, but should be defined by the scope of the accompanying claims.

## Claims

1. A microneedle formulation composition, comprising:
a biodegradable molecule;
a peptide-based drug;
an oil;
a stabilizer;
an antioxidant; and
a surfactant,
wherein the microneedle formulation composition is provided in a gel form.

2. The microneedle formulation composition according to claim 1, wherein the biodegradable polymer is comprised in a ratio of 5 wt.% to 20 wt.% based on the total weight of the composition.

3. The microneedle formulation composition according to claim 1, wherein the oil comprises at least one selected from the group consisting of castor oil, corn oil, olive oil, peanut oil, peppermint oil and soybean oil.

4. The microneedle formulation composition according to claim 1, further comprising at least one substance selected from disaccharides such as sucrose, lactose, maltose and trehalose, polysaccharides such as dextran and cellulose, and sugar alcohols such as mannitol and sorbitol as a stabilizer.

5. The microneedle formulation composition according to claim 1, wherein the peptide-based drug comprises a parathyroid hormone (PTH (1-34)).

6. The microneedle formulation composition according to claim 1, wherein the oil is comprised in a ratio of 0.1 wt.% to 5.0 wt.% based on a total weight of the composition.

7. The microneedle formulation composition according to claim 1, wherein the surfactant comprises at least one selected from the group consisting of polysorbate 20, polysorbate 40, polysorbate 80, sorbitan monolaurate 20 (Span 20), sorbitan monolaurate 80 (Span 80) and polyethylene glycol 400 (PEG 400).

8. The microneedle formulation composition according to claim 7, wherein the surfactant is comprised in a ratio of 0.01 wt.% to 5.0 wt.% based on the total weight of the composition.

9. The microneedle formulation composition according to claim 1, wherein the antioxidant comprises at least one selected from the group consisting of EDTA, L-methionine, ascorbic acid and L-glutamine.

10. The microneedle formulation composition according to claim 9, wherein the antioxidant is comprised in a ratio of 0.01 wt.% to 3.0 wt.% based on the total weight of the composition.

11. A microneedle, comprising:
a tip configured to supply an active ingredient into a subject and to have a shape extending in at least one direction; and
a base for supporting the tip,
wherein the tip comprises a biodegradable polymer; a peptide-based drug as an active ingredient; an oil; a stabilizer; an antioxidant; and a surfactant.

12. The microneedle according to claim 11, further comprising a guider provided inside the tip and configured to extend from the base toward the tip,
wherein, when the tip is inserted into a subject, the tip is separated from the guider.

13. The microneedle according to claim 11, wherein the peptide-based drug comprises a parathyroid hormone (PTH (1-34)),
wherein a content of the parathyroid hormone is 5 to 40 wt% based on a total weight of the tip.

14. The microneedle according to claim 11, further comprising a base holder provided on an opposite surface to a surface of the base, to which the base and the tip are bonded, to provide a bonding site to which the base and an outer member can be bonded.

15. The microneedle according to claim 11, wherein the biodegradable polymer is comprised in a ratio of 25 wt.% to 65 wt.% based on the total weight of the tip.
